# EUROPEAN PATENT APPLICATION

(11) **EP 2 417 964 A1**
(43) Date of publication of application: **15.02.2012**
(21) Application number: 10009163.6
(22) Date of filing: 03.09.2010
(51) Int. Cl.: A61K 8/42, A61Q 5/04, A61Q 5/08, A61Q 5/10

(54) **Method of decreasing cysteic acid formation in keratin fibres**

(71) Applicant: KPSS-Kao Professional Salon Services GmbH, 64297 Darmstadt (DE)
(72) Inventor: Schmelz, Sandra, 97828 Marktheidenfeld (DE); Meuser, Alexandra, Dr., 63329 Egelsbach (DE); Uellner, Martin, 64295 Darmstadt (DE)
(74) Representative: Grit, Mustafa

(57) **Abstract**

The present invention relates to a method of decreasing cysteic acid formation in keratin fibers especially human hair which is always resulting of a chemical treatment of hair such as permanent shaping and colouring and especially oxidative colouring. The method concerns inclusion of a diamide compound into the chemical treatment compositions. Accordingly, first object of the present invention is method of reducing cysteic acid formation in keratin fibers especially human hair during a chemical treatment process wherein hair is applied a composition comprising at least one diamide compound. Preferably, the diamide compound is selected from compounds according to the general structure 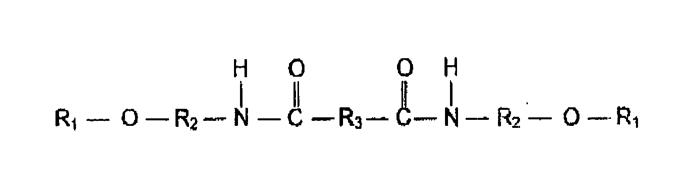 wherein R₁ is a linear or branched, saturated or unsaturated alkyl chain with 1 to 12 C atoms which may be substituted with hydroxy and/or alkoxy groups, preferably R₁ is linear or branched, saturated or unsaturated alkyl chain with 1 to 12 C atoms which may be substituted by 1 to 3 substituents selected from a hydroxy group and C₁ to C₆ alkoxy group, more preferably R₁ is a unsubstituted alkyl group with 1 to 12 C atoms, and alkyl group with 2 to 12 C atoms substituted by one or two hydroxy groups, by one alkoxy group with 1 to 6 C atoms or by one hydroxyl and one alkoxy group with 2 to 6 C atoms, R₂ is linear or branched alkyl chain with 1 to 5 C atoms, preferably linear or branched alkyl chain with 2 to 5 C atoms and more preferably an alkyl chain with 2 to 3 C atoms, and R₃ linear or branched, saturated or unsaturated alkyl chain with 1 to 22 C atoms, preferably linear or branched, saturated or unsaturated alkyl chain with 11 to 22 C atoms.

## Description

The present invention relates to a method of decreasing cysteic acid formation in keratin fibers especially human hair which is always resulting of a chemical treatment of hair such as permanent shaping and colouring and especially oxidative colouring The method concerns inclusion of a diamide compound into the chemical treatment compositions.

It is known that chemical treatment causes hair damages. Hair damage may be caused by using excessive alkaline and/or oxidative treatments. Hair treated with such conditions reportedly often loses its moisture, becomes less combable due to physical alterations of its surface structure and as a result hair feels unnatural because of loss of its natural softness, elasticity and shine.

Another aspect of the hair damage is chemical alteration of hair substance during chemical treatment which usually causes increase of cysteic acid concentration. There have been attempt to reduce cysteic acid formation in hair during chemical treatments, so far there are no reports known by the current inventors.

It has surprisingly been found out that a chemical treatment composition comprising at least one diamide compound reduces considerably formation of cysteic acid in keratin fibres.

The use of diamide compound is known in the art in chemical treatment compositions such as oxidative colouring and permanent shaping from US 2003/0208858 A1 and US 2003/0215416 A1, respectively. Both documents do not disclose anything on reducing cysteic acid formation in hair.

Accordingly, first object of the present invention is method of reducing cysteic acid formation in keratin fibers especially human hair during a chemical treatment process wherein hair is applied a composition comprising at least one diamide compound. Further object of the present invention is the use of diamide compound for reducing cysteic acid formation in keratin fibers especially human hair during chemical treatment of keratin fibers, especially human hair.

With the term diamide compound it is meant that a compound consisting of at least two amide groups In its molecule.

Preferably, the diamide compound is selected from compounds according to the general structure 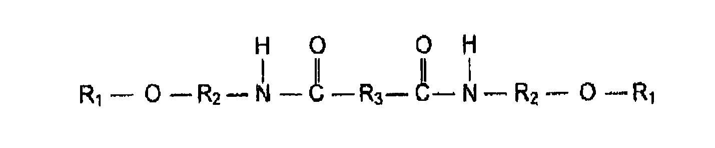 wherein R₁ Is a linear or branched, saturated or unsaturated alkyl chain with 1 to 12 C atoms which may be substituted with hydroxy and/or alkoxy groups, preferably R₁ is linear or branched, saturated or unsaturated alkyl chain with 1 to 12 C atoms which may be substituted by 1 to 3 substituents selected from a hydroxy group and C₁ to C₆ alkoxy group, more preferably R₁ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₂ is linear or branched alkyl chain with 1 to 5 C atoms, preferably linear or branched alkyl chain with 2 to 5 C atoms and more preferably an alkyl chain with 2 to 3 C atoms, and R₃ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

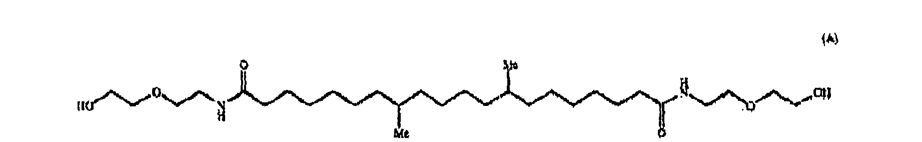

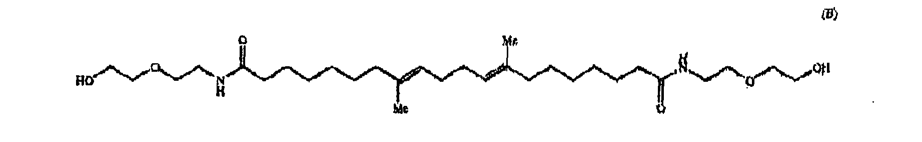

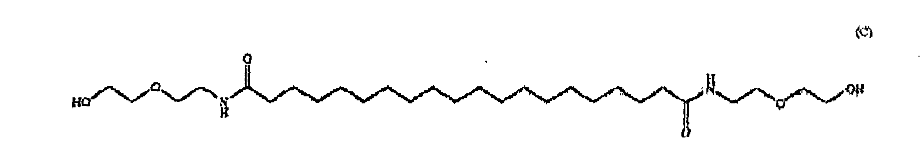

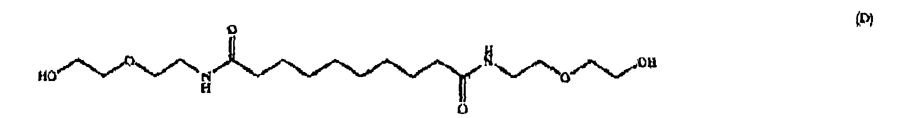

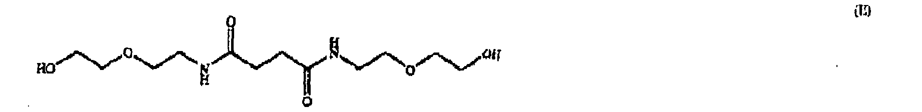

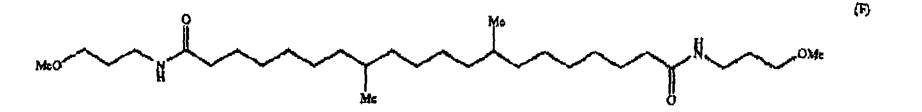

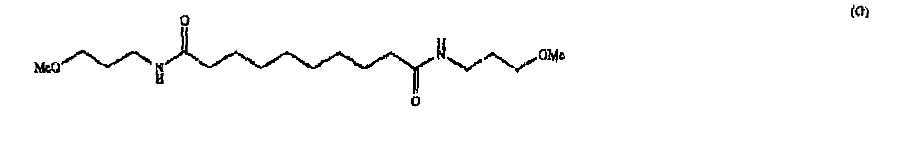

Particularly preferred diamide compound is the compound F which is bis (methoxypropylamido) isodocosane and commercially available from Kao Corporation - Japan.

Concentration of at least one diamide compound in the composition is in the range of 0.01 to 5% by weight calculated to total composition.

It has furthermore been found out that the diamide compound suppresses formation of cysteic acid when it is comprised in the chemical treatment composition. Chemical treatments here referred to are the ones usually carried out under alkaline and/or oxidizing conditions which are generally known to be permanent shaping, bleaching and colouring.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The permanent shaping compositions comprise at least one reducing agent at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 1.0 to 12.5%, more preferably 1.5 to 12.5%, most preferably 2.0 to 12.5% by weight, calculated to total composition as free thioglycolic acid as reference substance.

The compositions containing thioglycolates are customarily applied at a pH-value between 7 and 10, in particular 8.5 and 9.5.

After reduction of the disulfide bonds, an oxidizing agent is applied and processed in order to restore the disulfide bonds. It has been found out that cysteic acid formation is reduced when diamide compound is comprised in reducing composition and/or in oxidizing composition, preferably in oxidizing composition.

Therefore, present invention is furthermore on a method for reducing formation of cysteic acid in keratin fibers especially human hair wherein hair is washed or shampooed first and wound on the curlers, subsequently a reducing composition comprising at least one reducing agent is applied onto hair and after 1 to 45 min, preferably 1 to 30 min of processing time, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair wherein reducing and/or oxidizing compositions, preferably oxidizing composition, comprise at least one diamide compound.

As known from the permanent shaping art, additional composition known as intermediate treatment mainly comprising inorganic salt may optionally be applied between reducing and oxidizing steps and without rinsing it off, hair is further treated with oxidizing composition. The intermediate treatment composition has a pH value between 2.5 to 6, preferably 3 to 5.5 and most preferably 3 to 5. It is furthermore the object of the present invention that the diamide compound is comprised in the intermediate composition and applied onto hair in the above disclosed method after rinsing off the reducing composition and processed on the hair for a short period of time up to 10 min and without rinsing it off oxidizing composition is applied.

In the above method, curlers may also be removed after rinsing off the reducing agent and before applying the oxidizing agent.

Permanent shaping may also be carried out for the purpose of straightening hair. Further object of the present invention is method of reducing formation of cysteic acid in keratin fibers especially human hair wherein hair is washed and/or shampooed and dried and reducing composition comprising at least one reducing compound is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off wherein reducing and/or oxidizing compositions, preferably oxidizing composition, comprise at least one diamide compound.

As known in the art, bleaching compositions are prepared by mixing substantially anhydrous composition with an aqueous oxidizing composition. Substantially anhydrous composition can be in powder, especially dust free powder, suspension and paste forms. Substantially anhydrous composition comprises at least one compound with bleaching effect. Suitable compounds are in general peroxides. Useful as such are in particular persulfates such as sodium and potassium persulfate, ammonium persulfate, earth alkali peroxides such as magnesium peroxide, melamine peroxide or urea peroxide or phtholimidoperoxyhexanoic acid, and mixtures thereof. The proportion of peroxides is at least 5%, preferably in the range of 20 to 80%, more preferably 25 to 60% and most preferably 30 to 55% by weight, calculated to total of each composition. Substantially anhydrous composition can also comprise 0.1% to 10% by weight, calculated to total of each composition, at least one ammonium salt. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixture or ammonium salts. The total proportion of the compounds with bleaching effect preferably ranges from 5% to 85%, preferably 20% to 80%, more preferably 25 to 70% and most preferably 30 to 60% by weight calculated to total of each composition.

For the purpose of bleaching, oxidizing agents suitable are same as the ones mentioned suitable for permanent shaping.

According to the present invention, method of reducing formation of cysteic acid wherein an anhydrous composition comprising at least one compound with bleaching effect is mixed with an aqueous composition comprising at least one oxidizing agent is applied onto hair and left on the hair for 1 to 45 min and rinsed off from hair wherein one of the compositions mixed or both compositions and preferably oxidizing composition comprises at least one diamide compound.

Colouring especially oxidative colouring hair is carried out by mixing two compositions wherein the first comprises at least one oxidative dyeing agent and the second comprises at least one oxidizing agent.

Some examples to the oxidative dyeing precursors are p-phenylenediamine, p-methylaminophenol and substituted p-phenylenediamines such as 2,5-diamino-toluene, 2-n-propyl or 2-ethyl-p-phenylenediamine, 2,6-di-methyl-p-phenylenediamine, 2-(2,5-diaminophanyl) ethanol, 1-amino-4-bis-(2'-hydroxyethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobranzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene. 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and 1,2,4-triamino benzene, or the water-soluble salts thereof.

Total concentration of oxidative dye precursors is in the range of 0.01 to 10% by weight, preferably 0.05 to 7.5% by weight and more preferably 0.1 to 5% by weight, calculated to total composition prior to mixing with an oxidizing agent.

The oxidative dyeing composition preferably comprises one or more coupling substances. Suitable ones are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 1,3-bis(2,4-diaminophenoxy) propane and/or its respective salts 2-dimethyl-amino-5-aminopyridine, 2,6-diaminopyridine, 5-amino-2-methylphenol, 2-methyl-5-hydroxyethylaminophenol, 2-methyl-5-amino-6-chlorphenol, 2-bis(2-hydroxyethyl)aminotoluene, 2-amino-5-methylphenol,α-naphthol, 4,6-dichlororesorcinol, 1-hydroxy naphthalene, 4-hydroxy-1,2-methylenedioxy benzene, 1,5-dihydroxy naphthalene, 1,6-dihydroxy naphthalene, 1,7-dihydroxy naphtalene, 2,7-dihydroxy naphthalene, 1-hydroxy-2-methyl naphthalene, 4-hydroxy-1.2-methyldioxybenzene, 5-amino-2-methoxyphenol, hydroxybenzomorpholine, 1,2,4-trihydroxybenzene, phenylmethylpyrazolone, 3-amino-2,4-dichlorophenol, hydroxyethyl-3,4-methylenedioxyaniline, 2.6-dimethoxy-3,5-dimethylpyridine, 5-amino-4-chloro-2-methylphenol, hydroxypropyl-bis-(N-hydroxyethyl-p-phenylenediamine), 1-acetoxy-2-methylnaphthalene, 2,2'-methylenebis-4-aminophenol and/or or their respective salts.

Total concentration of one or more coupling agent is in the range of 0.001 to 5% by weight, preferably 0.005 to 3% by weight and more preferably 0.01 to 2.5% by weight, calculated to total composition prior to mixing with an oxidizing agent.

Oxidative dyeing compositions may further comprise one or more direct dyes. Direct dyes are found to be useful for adjusting colour tone within the meaning of the present invention.

Direct dyes suitable are cationic, anionic and/or nitro dyes. Suitable non-limiting examples to cationic ones are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green, Basic Orange 31, 1, Basic Red 2, Basic Red 12 Basic Red 22, Basic Red 76 Basic Red 51, Basic Violet 1, Basic Violet 2, Basic Violet 3, Basic Violet 10, Basic Violet 14, Basic Yellow 57 and Basic Yellow 87.

Suitable non-limiting examples to anionic ones are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Suitable non-limiting examples to nitro dyes are HC Blue No.2, HC Blue No.4, HC Blue No.5, HC Blue No.6, HC Blue No.7, HC Blue No.8, HC Blue No.9, HC Blue' No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid. Preferred direct dyes are the nitro dyes.

Total concentration of one or more direct dyes is in the range of 0.001 to 10% by weight calculated to total composition prior to mixing with oxidizing agent.

Method of reducing formation of cysteic acid in keratin fibers especially human hair wherein a composition comprising at least one oxidative dye precursor and optionally at least one coupling substance and optionally at least one direct dye is mixed with an aqueous composition comprising at least one oxidizing agent is mixed immediately before applying onto hair and applied onto hair and processed for up to 45 min and rinsed off from hair and hair is optionally washed optionally with a shampoo composition and optionally dried, wherein at least one of the two compositions mixed and/ or both compositions, preferably aqueous composition comprising at least one oxidizing agent comprises at least one diamide compound.

Oxidizing agents suitable for colouring compositions are same as the ones mentioned above for permanent shaping method.

It should be noted that pH of compositions comprising at least one oxidative dye precursor and optionally at least one coupling substance and optionally at least one direct dye is in the range of 6 to 12, preferably 6.5 to 11 and especially 6.8 to 10, prior to mixing with an aqueous composition comprising at least one oxidizing agent.

pH of aqueous composition comprising at least one oxidizing agent is in the range of 2 to 6, preferably 2 to 5 and especially 2 to 4, prior to mixing with dyeing composition.

pH of the ready to use oxidative colouring composition is in the range of 6 to 12, preferably 6.5 to 11 and especially 6.8 to 10.

Permanent shaping and colouring compositions can be in the form of solutions, dispersions, gels and emulsions. Anhydrous bleaching composition may be powder, granulate, tablet, dispersion, suspension, cream and or paste forms. It should be noted that the form of the preparation does not have an influence on the effect of the invention.

Compositions suitable for carrying out the method and use according to the present invention can comprise additionally substances customarily found in any form and purpose disclosed below, unless specifically mentioned for one type of composition,

Colouring composition of present invention can comprise additionally in the base formulation fatty acids with 0 to 3 ethylenic bonds and with fatty acyl chain length of 12 to 22 C atoms. Concentration of the fatty acids can be in the range of 0.1 to 10%, preferably 0.1 to 7.5% and most preferably 0.2 to 5% by weight calculated to the total composition, prior to mixing with oxidizing agent. Non-limiting examples are myristic acid, palmitic acid, behenic acid, stearic acid, oleic acid, linoleic acid. The most preferred fatty acid is oleic acid.

Compositions especially the ones in emulsion form comprise at least one fatty alcohol or mixture of fatty alcohols with the chain length of 14 to 22 C atoms which may be straight or branched, saturated or unsaturated. Examples to suitable fatty alcohols, without limiting the choice, are myristyl alcohol, cetyl alcohol, stearyl alcohol, behenyl alcohol, oleyl alcohol and cetostearyl alcohol, octyldodecanol. The most preferred is cetostearyl alcohol well known with its trade name Lanette O or as Lanette N in mixture with sodium cetearyl sulfate from Cognis. Total fatty alcohol content should be in the range of 1 to 20% by weight, calculated to total composition prior to mixing with an oxidizing agent.

Compositions can comprises surfactants selected from anionic, amphoteric (or zwiterionic) and/or cationic surfactants as emulsifier or solubilizer. Cationic surfactants are as well used as hair conditioners.

The non-ionic surfactants are ethoxylated fatty alcohols with an alkyl chain of 12 to 24 C atoms and with number of ethoxyl groups of 2 to 50, preferably 10 to 30. Examples are ceteth-20, seteareth-30, palmeth-20, steareth -20, beheneth-20 etc. These compounds are named according to the fatty alcohol they are originating and number of ethoxyl groups is given at the end These compounds are well known emulsifiers and found in any cosmetic ingredient book.

Further suited nonionic surfactants are, especially in mixture with fatty alcohol ethoxylates, for example, long-chain fatty acid mono- and dialkanolamides, such as coco fatty acid mono- or diethanolamide and myristic fatty acid mono or diethanolamide, stearic acid mono or diethanolamide.

Further nonionic surfactants suited again especially in admixture with fatty alcohol ethoxylates mentioned above are alkyl polyglucosides of the general formula

R₆-O-(R₃O)ₙ-Zₓ,

wherein R₆ is an alkyl group with 8 to 18 carbon atoms, R₃ is an ethylene or propylene group, Z is a saccharide group with 5 to 6 carbon atoms, n is a number from 0 to 10 and x is a number between 1 and 5.
Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}". "Aromox^{®}" or "Genaminox^{®}".

Anionic surfactants suitable within the scope of the invention are in principal known from the cleansing compositions and may be present in an amount from 0.1 to about 10% by weight, calculated to the total composition prior to mixing with an oxidizing agent. Compatibility of anionic surfactant in the composition should be taken into account when choosing the type and the concentration.

These are anionic surfactants of the sulfate, sulfonate, carbonate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₅- (C₂H₄O)ₙ- O- CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 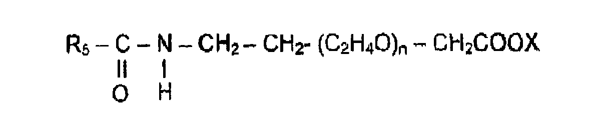 wherein R₅ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially referred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants in a mixture.

An overview of the anionic surfactants suitable for the present invention can furthermore be found in the monography of K. Schrader, "Grundlagen und Rezepturen der Kosmetika", 2nd Ed.(1989, Hüthig Buchverlag), pp. 595-600 and pp. 683 to 691.

As further surfactant component, the colouring compositions according to the invention can also contain amphoteric or zwitterionic surfactants, for examples in an amount from about 0.5 % to about 5 %, preferably from about 1 % to about 2.5 % by weight, calculated to the total composition.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacotate, cocoaminopropionate and sodium cocoamphopropionate and -acetate have also proven suitable.

Compositions may also comprise cationic surfactants as emulsifier, solubilizer and/or conditioning ingredients according to the formula 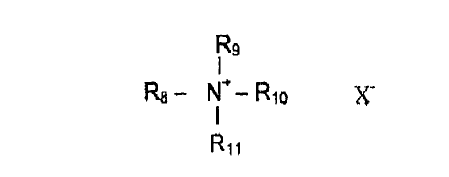 where R₈ is a saturated or unsaturated, branched or non-branched alkyl chain with 8-22 C atoms or

R₁₂ CO NH (CH₂)ₙ

where R₁₂ is saturated or unsaturated, branched or non-branched alkyl chain with 7-21 C atoms and n has typical value of 1 - 4 or

R₁₃ CO O (CH₂)ₙ

where R₁₃ is saturated or unsaturated, branched or non-branched alkyl chain with 7-21 C atoms and n has typical value of 1 - 4, and
R₉ is H or unsaturated or saturated, branched or non-branched alkyl chain with 1 - 22 C atoms or

R₁₂ CO NH (CH₂)ₙ

or

R₁₃ CO O (CH₂)ₙ

where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Form the above mentioned surfactants preferred are non-ionic and anionic surfactants and their mixtures.

Total surfactant concentration is in the range of 0.5 to 15%, preferably 1 to 10%, more preferably 1 to 7.5% by weight calculated to total composition.

Compositions may also comprise cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Further suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, polyquaternium 36, Polyquaternium 46. Among those the most preferred one is the Polyquaternium 11 as well known with its trade name Gafquat from ISP and as Luviquat PQ from BASF.

Typical concentration range for any of the cationic, conditioners mentioned above can be 0.01 - 5% by weight, preferably 0.03 - 2.5% by weight and more preferably 0.05 -1.5% by weight.

Compositions may comprise an organopolysiloxane wherein at least one silicium atom is linked to an alkylene group having a hetero-atom, in particular a nitrogen atom, with a poly-(N-acyl alkyleneimine) units of the formula 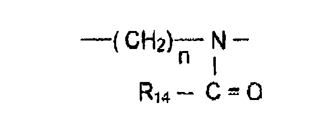 wherein n is a number from 1 to 5 and R₁₄ is hydrogen, a C₁-C₁₂₋alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxanelpolyethyl oxazoline copolymers of the formula 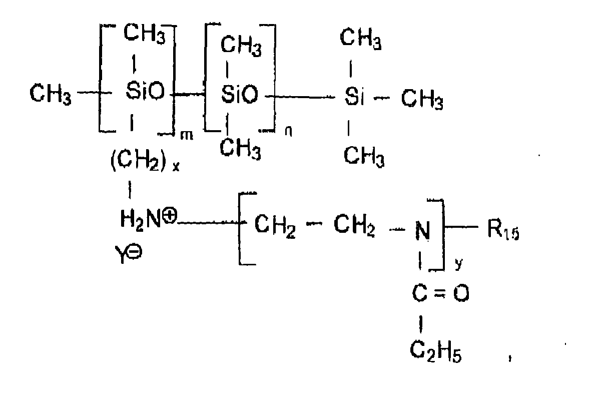 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Compositions may comprise organic solvents as penetration enhancers and also as a solubilizer. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents may be in the range from 0.5% to 20%, preferably 0.5 - 15%, more preferably 0.5 - 10%, by weight calculated to the total composition.

Compositions may comprise thickening agents. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1-2% by weight calculated to the total composition prior to mixing with oxidizing composition and depending on the desired consistency thereof.

Compositions may comprise further hair conditioning agents such as silicone oils either volatile or non-volatile, natural and synthetic oils. Among silicone oils those can be added to the compositions include dimethicone, dimethiconol. polydimethylsiloxane, DC fluid ranges from Dow Corning, arylated silicones such as phenyl trimethicone, natural oils such as olive oil, almond oil, avocado oil, wheatgerm oil, ricinus oil and the synthetic oils, such as mineral oil, isopropyl myristate, palmi-tate, stearate and isostearate, oleyl oleate, isocetyl stearate, hexyl laurate, dibutyl adipate, dioctyl adipate, myristyl myristate and oleyl erucate.

Additionally Polyols may be comprised in the compositions. Suitable polyols are such as glycerin, glycol and derivatives, polyethyleneglycoles known with trade names Carbowax PEG from Union Carbide and Polyox WSR range from Amerchol, polyglycerin and polyethyleneglycol mono or di fatty acid esters.

Compositions may further comprise at least one ubiquinone of the formula 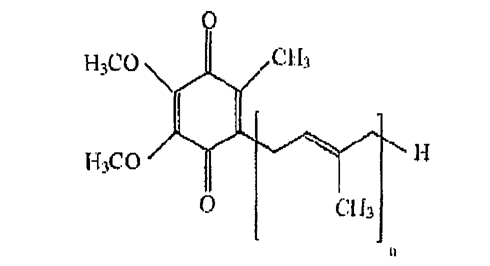 where n is a number between 1 and 10 at a concentration of 0.0001 to 1 %, preferably from 0.0002 to 0.75%, more preferably from 0.0002 to 0.5% and most preferably from 0.0005 to 0.5% by weight, calculated to total composition, prior to mixing with oxidizing composition.

The composition comprises ubiquinone which is preferably selected from the ones where n is a number between 6 and 10 and more preferably it is ubiquinone 50 where n is 10, also known as Coenzyme Q10.

Composition can comprise at least one amino acid. At least one amino acid is comprised at a concentration of 0.01 to 10%, preferably 0.05 to 7.5% and more preferably 0.1 to 5% and most preferably 0.25 to 5% by weight calculated to total of each composition.

Suitable amino acids are glycin, histidine, citrullin, asparagine, alanin, valin, leucin, isoleucin, pyrolin, tryptophane, phenylalanine, methionine, serine, tyrosine, threonine and gluatamine. Preferably, the amino acid is selected from glycin, histidine, citrullin, asparagine, alanin, valin, leucin, isoleucin, pyrolin, serine, tyrosine, threonine and gluatamine. More preferably, at least one amino acid is selected from glycin, histidine, asparagine, alanin, valin, leucin, pyrolin, serine, tyrosine and gluatamine, and most preferably at least one amino acid is selected from glycin, asparagine, alanin, valin, leucin, and serine.

Compositions may comprise further ceramide type of compound with the general formula 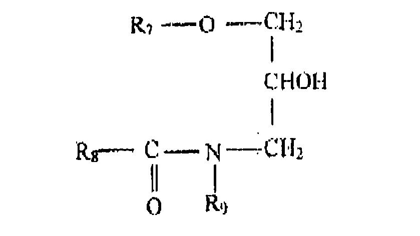 where R₇ and R₈ are independent from each other alkyl- or alkenyl group with 10 to 22 carbon atoms, R₉ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide.

Further optional ingredient are sterols, especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol, Among those phytosterols, the ones found in "Avocadin" which is the unsaponified fraction of the avocado oil is more preferred.

Compositions of the present invention can further comprise ingredients customarily found in such compositions such as alkalizing agents, preservatives antioxidants, fragrances and chelating agents.

The concentration of cysteic acid in hair is determined according to the method of Speakman, Stein und Moore (Anal. Chem., 30, 1190 (1958)) after hydrolyzing hair in 6M hydrochloric acid for 24 h at 110°C under vacuum and subsequently removing hydrochloric acid with a rotation evaporator.

The following examples are to illustrate the invention, but not to limit it.

### Example 1:

| **Hair colouring composition** | |
|---|---|
| | % by weight |
| Octyldodecanol | 1.3 |
| Cetearyl alcohol | 1.0 |
| Oleyl alcohol | 2.6 |
| Sodium lauryl sulphate | 1.0 |
| Xanthan gum | 1.0 |
| Sodium sulfite | 0.5 |
| Ascorbic acid | 0.5 |
| Toluene 2,5-diamine sulfate | 1.3 |
| m-aminophenol | 0.1 |
| Resorcinol | 0.4 |
| Tetrasodium EDTA | 0.2 |
| Fragrance, preservative | q.s |
| Ammonia 25% | 8.0 |
| Water | q.s. to 100 |

The above composition was mixed with an oxidizing composition comprising 6% by weight hydrogen peroxide and 0.1% by weight bis (methoxypropylamido) isodocosane at a weight ratio of colouring composition to oxidizing compositlon 1:1 and applied onto hair and processed 30 min at ambient temperature and rinsed off from hair.

For comparative purposes, oxidizing composition without bis (methoxypropylamido) isodacosane was used.

Following cysteic acid concentrations were found:

| | Cysteic acid concentration (mol%) |
|---|---|
| Untreated hair | 0.72 |
| Coloured with Example 1 | 0.75 |
| Coloured with comparative composition | 0.97 |

For the above results it is clear that incorporation of a diamide, bis (methoxypropylamido) isodocosane, reduced the formation of cysteic acid resulting from colouring hair with a composition comprising otherwise no diamide.

### Example 2

| **Permanent waving composition** | | |
|---|---|---|
| Ammonium thioglycolate (60%) | | 21.3 (% by wt.) |
| Ammonium hydrogen carbonate | | 5.0 |
| 1,3- butylene gylcol | | 3.0 |
| Bis (methoxypropylamido) isodocosane | | 0.05 |
| Polyquaternium-6 | | 0.2 |
| PEG-40-Hydrogenated castor oil | | 0.7 |
| Perfume | | 0.4 |
| Ammonia, 25% | ad pH | 8.3 |
| Water | ad | 100.0 |

With this composition the hair was permanently waved. First the above composition was applied and processed for about 15 minutes, rinsed and neutralized for about 8 minutes with a customary 2.5% H₂O₂ composition. Homogeneous wave appearance was obtained.

It was found out that hair permanently shaped with the above composition contained less cysteic acid that the same composition but not comprising diamide compound.

Furthermore, it was found out that when diamide compound was comprised in the oxidizing agent the level of cysteic acid was further reduced.

### Example 3

| **Anhydrous bleaching composition** | |
|---|---|
| | % by weight |
| Potassium persulfate | 40 |
| Sodium persulfate | 5 |
| Sodium carbonate | 1 |
| Sodium silicate | 10 |
| Diatomaceous Earth | 43 |
| Polyquaternium-67 | 1 |

The above composition is prepared by combining all powder components together and mixing until homogeneity in a suitable mixer.

The above composition was mixed with an oxidizing composition of the following composition at a weight ratio of water free powder to oxidizing lotion 1:2 prior to application onto hair.

| **Oxidizing Composition** | |
|---|---|
| | % by weight |
| Hydrogen peroxide | 9.0 |
| Phosphoric acid | 0.5 |
| Sodium lauryl sulphate | 0.2 |
| Bis (methoxypropylamido) isodocosane | 0.1 |
| Disodium hydrogen phsophate | 0.1 |
| Salicylic acid | 0.1 |
| Water | to 100 |

The pH of the above composition was 3.0.

The mixture of powder and oxidizing composition had a pH of 9.5.

Bleaching was carried out by applying the mixed aqueous composition onto hair and after processing for approximately 20 min at ambient temperature, it was rinsed off from hair.

In the same way, a composition without Bis (methoxypropylamido) isodocosane was prepared and hair was bleached with that composition in the same way as above.

It was observed that hair bleached with the composition comprising bis (methoxypropylamido) isodocosane contained much less cysteic acid compared to the hair bleached with a composition not comprising a diamide compound.

## Claims

1. Method of reducing cysteic acid formation in keratin fibers especially human hair during a chemical treatment process **characterized in that** hair is applied a composition comprising at least one diamide compound.

2. A method for reducing formation of cysteic acid in keratin fibers especially human hair **characterized in that** hair is washed or shampooed first and wound on the curlers, subsequently a reducing composition comprising at least one reducing agent is applied onto hair and after 1 to 45 min, preferably 1 to 30 min of processing time, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair wherein reducing and/or oxidizing compositions, preferably oxidizing composition, comprise at least one diamide compound.

3. The method according to claim 2 **characterized in that** hair is applied another composition comprising at least one inorganic salt and optionally comprising at least one diamide compound after rinsing off the reducing composition and prior to application of the oxidizing composition having a pH without rinsing off oxidizing composition is applied.

4. The method according to claims 2 and 3 **characterized in that** curlers are removed from hair immediately after rinsing off the reducing composition.

5. Method of reducing formation of cysteic acid in keratin fibers especially human hair **characterized in that** hair is washed and/or shampooed and dried and reducing composition comprising at least one reducing compound is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, Is applied onto hair and left on the hair 1 to 20 min and rinsed off wherein reducing and/or oxidizing compositions, preferably oxidizing composition, comprise at least one diamide compound.

6. Method of reducing formation of cysteic acid in keratin fibers especially human hair **characterized In that** a composition comprising at least one oxidative dye precursor and optionally at least one coupling substance and optionally at least one direct dye is mixed with an aqueous composition comprising at least one oxidizing agent is mixed immediately before applying onto hair and applied onto hair and processed for up to 45 min and rinsed off from hair and hair is optionally washed optionally with a shampoo composition and optionally dried, wherein at least one of the two compositions mixed and/ or both compositions, preferably aqueous composition comprising at least one oxidizing agent comprises at least one diamide compound.

7. The method according to claim 6 **characterized in that** composition comprising at least one oxidative dye precursor is aqueous composition.

8. The method according to claims 6 and 7 **characterized in that** composition comprising at least one oxidative dye precursor is aqueous emulsion composition and comprises further at least one fatty alcohol and at least one emulsifier.

9. Method of reducing formation of cysteic acid in keratin fibers especially human hair **characterized in that** an anhydrous composition comprising at least one compound with bleaching effect is mixed with an aqueous composition comprising at least one oxidizing agent, preferably hydrogen peroxide, is applied onto hair and left on the hair for 1 to 45 min and rinsed off from hair wherein one of the compositions mixed or both compositions and preferably oxidizing composition comprises at least one diamide compound.

10. The method according to claim 9 **characterized in that** anhydrous composition comprises at least one compound with bleaching effect at a concentration of at least 5% by weight calculated to total composition.

11. The method according to any of the preceding claims **characterized in that** diamide compound is present at a concentration of 0.01 to 5% by weight calculated to total composition prior to mixing.

12. The method according to any of the preceding claims **characterized in that** at least one diamide compound is selected from compounds according to general structure 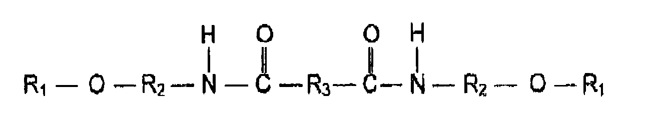 wherein R₁ is a linear or branched, saturated or unsaturated alkyl chain with 1 to 12 C atoms which may be substituted with hydroxy and/or alkoxy groups, R₂ is linear or branched alkyl chain with 1 to 5 C atoms, and R₃ linear or branched, saturated or unsaturated alkyl chain with 1 to 22 C atoms.

13. The method according to any of the preceding claims **characterized in that** at least one diamide compound is selected from

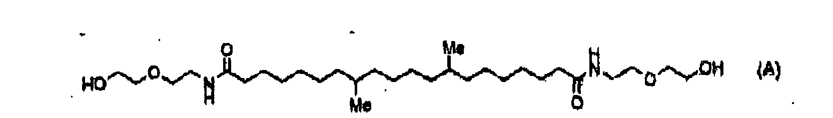

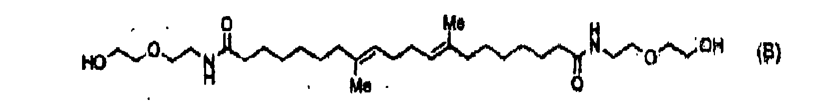

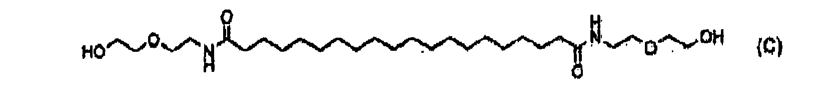

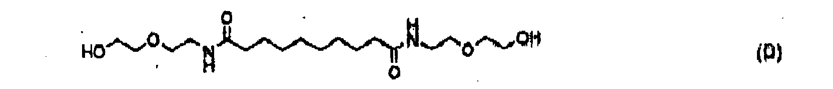

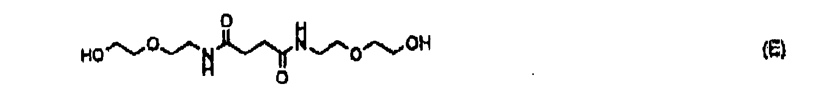

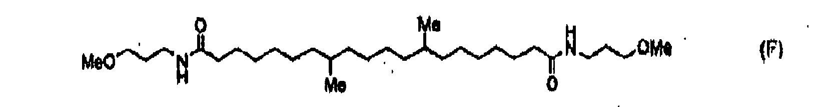

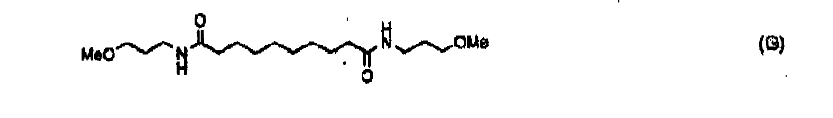

14. The method according to any of the preceding claims **characterized in that** at least one diamide compound is

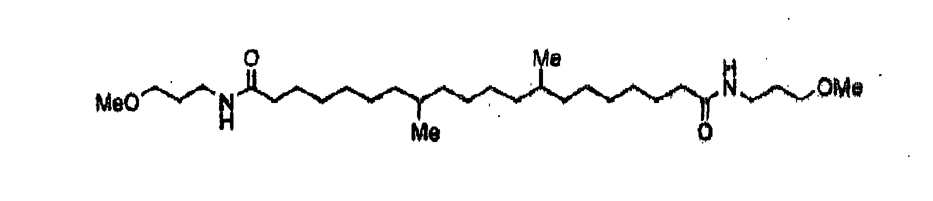

15. Use of at least one diamide compound in compositions for bleaching, permanent shaping and colouring keratin fibres especially human hair for reducing cysteic acid formation.
